# EUROPEAN PATENT APPLICATION

(11) **EP 4 506 683 A2**
(43) Date of publication of application: **12.02.2025**
(21) Application number: 24192458.8
(22) Date of filing: 01.08.2024
(51) Int. Cl.: G01N 23/04, G21F 5/015, G21G 4/06

(54) **METHODS AND APPARATUS FOR CONTROL OF RADIOGRAPHIC SOURCE EXPOSURE**

(30) Priority: 11.08.2023 US 202363518995 P; 26.07.2024 US 202418785917
(71) Applicant: Illinois Tool Works Inc., Glenview IL 60025 (US)
(72) Inventor: WOOD, David, Glenview, 60025 (US)
(74) Representative: HGF

(57) **Abstract**

Example remote controls for radiographic sources include: a drive cable configured to detachably couple to a radiographic source via a connector; a drive gear configured to advance the drive cable in a first direction toward the connector and to retract the drive cable in a second direction away from the connector; a control conduit configured to guide a portion of the drive cable located between the drive gear and a radiographic source housing; and a drive cable storage drum on an opposite side of the drive gear from the control conduit, and configured to store a portion of the drive cable.

## Description

### RELATED APPLICATIONS

The present application claims the benefit of U.S. Provisional Patent Application Serial No. 63/518,995, filed August 11, 2023, entitled "METHODS AND APPARATUS FOR CONTROL OF RADIOGRAPHIC SOURCE EXPOSURE." The entirety of U.S. Provisional Patent Application Serial No. 63/518,995 is expressly incorporated herein by reference.

### FIELD OF THE DISCLOSURE

This disclosure relates generally to radiography and, more particularly, to methods and apparatus for control of radiographic source exposure.

### BACKGROUND

Industrial radiography is often used for producing images of objects that are otherwise difficult to inspect, and involves exposing a source of high-energy radiation (e.g., gamma rays) and collecting penetrating and/or reflected rays to form a radiographic image. When not in use, gamma ray sources, such as radioactive isotopes, are stored in shielding devices.

### SUMMARY

Methods and apparatus for control of radiographic source exposure are disclosed, substantially as illustrated by and described in connection with at least one of the figures, as set forth more completely in the claims.

### BRIEF DESCRIPTION OF THE DRAWINGS

These and other features, aspects, and advantages of the present disclosure will become better understood when the following detailed description is read with reference to the accompanying drawings in which like characters represent like parts throughout the drawings, wherein:
FIGS. 1A and 1B illustrate example radiographic system for providing radiation for radiography, in accordance with aspects of this disclosure.
FIG. 2 illustrates an example radiography remote control including a storage drum for storing a portion of a control cable, in accordance with aspects of this disclosure.
FIG. 3 is a cross-sectional view of the example remote control of FIG. 2.
FIG. 4 is a flowchart representative of an example method which may be performed to control exposure of a radiographic source using the remote control of FIGS. 2 and 3.
FIG. 5 is a flowchart representative of an example method which may be performed to clean the control cable of the remote control of FIGS. 2 and 3.

The figures are not necessarily to scale. Wherever appropriate, similar or identical reference numerals are used to refer to similar or identical components.

### DETAILED DESCRIPTION

For the purpose of promoting an understanding of the principles of the claimed technology and presenting its currently understood, best mode of operation, reference will be now made to the embodiments illustrated in the drawings and specific language will be used to describe the same. It will nevertheless be understood that no limitation of the scope of the claimed technology is thereby intended, with such alterations and further modifications in the illustrated device and such further applications of the principles of the claimed technology as illustrated therein being contemplated as would typically occur to one skilled in the art to which the claimed technology relates.

Conventional radiography remote controls have multiple conduits to manage the control cable. A first conduit guides a control cable between the drive gear of the remote control and the radiographic source housing, while a second conduit provides a path for portions of the excess length of the control cable (e.g., the part of the control cable which is on an opposite side of the drive gear from the first conduit). Conventional remote control devices may bond the first and second conduits for ease of manipulation. However, the bonded conduits may reduce the flexibility of the remote control and/or the control cable, and/or may reduce the length of control cable which is usable for a given length of conduit.

Disclosed example remote controls for radiography remove the second conduit and store the excess length of the control cable in a storage drum or other storage container. In some examples, advancement of the control cable toward the radiography source pulls the storage cable from the storage drum, while retraction of the control cable from the radiography source causes the control cable to be wound or otherwise stored within the storage drum. Disclosed example remote controls may have a reduced weight and/or improved flexibility compared to conventional remote controls, and/or may allow for a longer control cable to be used for a given length of control conduit. In some examples, the storage drum may be used for easily cleaning and/or relubricating the control cable in accordance with maintenance procedures.

Disclosed example remote controls for radiographic sources include: a drive cable configured to detachably couple to a radiographic source via a connector; a drive gear configured to advance the drive cable in a first direction toward the connector and to retract the drive cable in a second direction away from the connector; a control conduit configured to guide a portion of the drive cable located between the drive gear and a radiographic source housing; and a drive cable storage drum on an opposite side of the drive gear from the control conduit, and configured to store a portion of the drive cable.

In some example remote controls, the drive cable storage drum is a fixed size and a fixed position with respect to the drive gear. In some example remote controls, the drive cable storage drum is configured to coil the drive cable within the drive cable storage drum as the drive cable is retracted by the drive gear. In some example remote controls, the drive cable storage drum includes a window configured to enable viewing of at least a portion of the drive cable within the drive cable storage drum.

Some example remote controls further include a drive gear housing configured to house the drive gear, in which the drive gear housing has a grip. In some example remote controls, the drive cable storage drum is coupled to the drive gear housing via the grip. Some example remote controls further include a conduit connector configured to secure the control conduit to the radiographic source housing.

Some example remote controls include a motor configured to actuate the drive gear. Some example remote controls include a handle configured to actuate the drive gear. Some example remote controls include a housing configured to house the drive gear, in which the control conduit is configured to rotate with respect to the housing. Some example remote controls include a storage conduit positioned between the drive gear and the drive cable storage drum.

Disclosed example methods to actuate a radiographic source involve: actuating a remote control coupled to a radiographic source in a first direction to extend a portion of a source cable of the remote control from a storage drum toward the radiographic source; and actuating the remote control in a second direction to retract a portion of the source cable of the remote control into the storage drum.

Some example methods further involve cleaning at least a portion of the source cable in the storage drum. Some example methods further involve connecting the source cable to the radiographic source and locking a conduit for the source cable to a housing of the radiographic source via a connector prior to extending the portion of the source cable. Some example methods further involve unlocking the conduit and detaching the source cable from the radiographic source after fully retracting the source cable.

Disclosed example methods to clean a remote control for a radiographic source involve: retracting a portion of a source cable of a remote control for a radiographic source into a storage drum attached to the remote control; and adding a cleaning agent to the storage drum to clean the portion of the source cable within the storage drum.

In some example methods, the cleaning agent comprises a solvent. Some example methods further involve removing the cleaning agent from the storage drum. Some example methods further involve adding a lubricant to the storage drum to lubricate the source cable. In some example methods, the retracting of the portion of the source cable involves turning a handle of the remote control in a retraction direction.

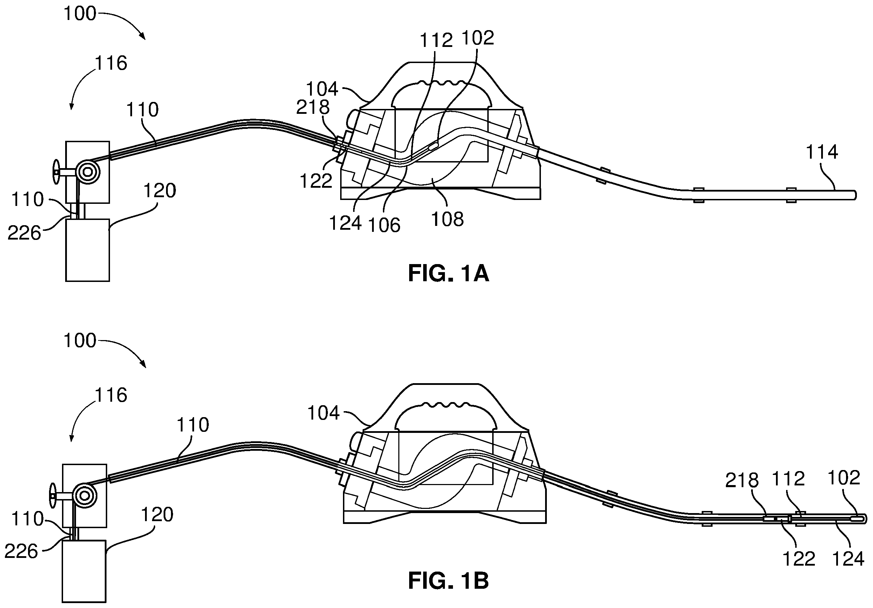

FIGS. 1A and 1B illustrate example radiographic system 100 for providing radiation for radiography. The radiographic system 100 of FIG. 1 includes a radiographic source 102 which is contained within a radiographic source housing 104. The example radiographic source 102 is a mass of radioactive material which emits radiation (e.g., X-rays and/or gamma rays) due to decay of the material.

The radiographic source housing 104 includes an S-shaped source tube 106 within a shield 108. The source tube 106 provides a pathway for the radiographic source 102 to be exposed to an exterior of the shield 108 and retracted to a shielded position within the interior of the shield 108. FIG. 1A illustrates the radiographic source 102 in the shielded position, and FIG. 1B illustrates the radiographic source in an exposed position.

To control the position of the radiographic source 102, the radiographic source housing 104 enables connection of a drive cable 110 to the radiographic source 102 for exposure and retraction of the radiographic source 102. The drive cable 110 may be physically attached or connected to a pigtail connector 112 that is physically coupled to the radiographic source 102.

When engaged, the drive cable 110 is controlled to extend into and through the source tube 106 to push the radiographic source 102 to an exposed position external to the radiographic source housing 104. Conversely, the drive cable 110 is retracted to pull the radiographic source 102 from the exposed position back into the source tube 106 to the shielded position, at which time the drive cable 110 may be detached from the radiographic source 102.

In the system 100 of FIG. 1, the exposed position of the radiographic source 102 may be controlled by a guide tube 114, through which the radiographic source 102 travels as the source 102 is pushed by the drive cable 110. The drive cable 110 has sufficient column strength to push the radiographic source 102 through the source tube 106 and through the guide tube 114. The remote control 116 physically engages the drive cable 110 to advance or retract the drive cable 110 relative to the remote control 116.

The example drive cable 110 is a flexible steel cable with a helical outer winding. The helical outer winding enables the remote control 116 to engage the drive cable 110 and advance and retract the drive cable 110 with a high degree of precision. A control conduit 118 guides the drive cable 110 between the remote control 116 and the radiographic source housing 104, to which the control conduit 118 is connected.

As disclosed in more detail below, the excess length of the drive cable 110 (e.g., the portion of the drive cable 110 that is on an opposite or storage side of the remote control 116 from the control conduit 118) may be stored in a storage drum 120 or other type of storage container. The example storage drum 120 is attached to the remote control 116, but may be attached via another conduit to reduce a carrying weight of the remote control 116.

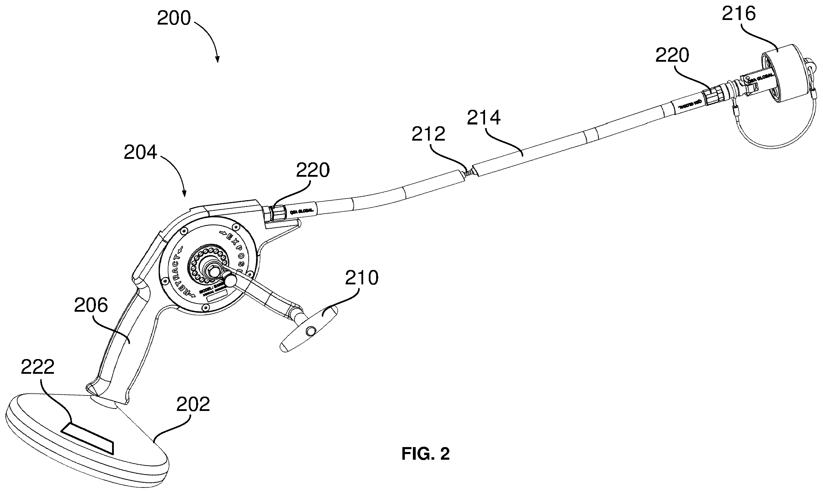

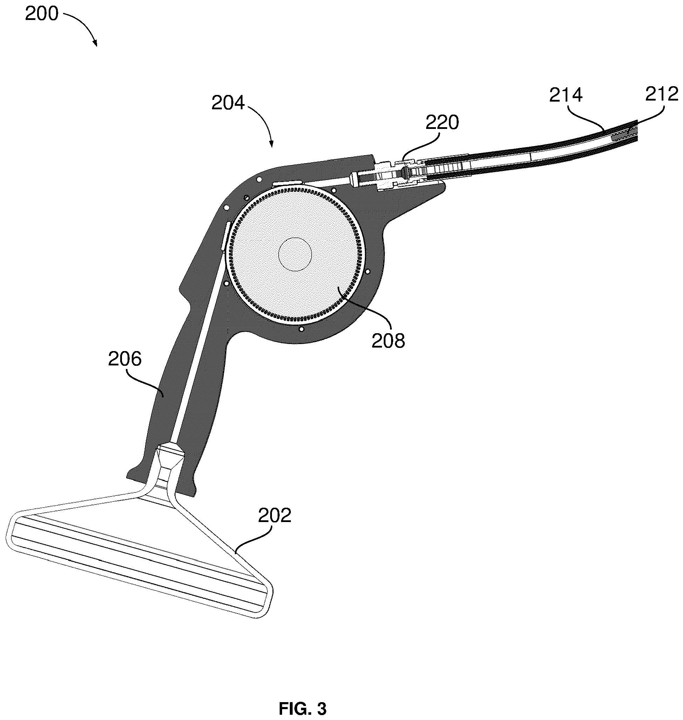

FIG. 2 illustrates an example radiography remote control 200 including a storage drum 20 for storing a portion of a control cable. FIG. 3 is a cross-sectional view of the example remote control 200 of FIG. 2. The example remote control 200 may implement the remote control 116, and the storage drum 202 may implement the storage drum 120 of FIG. 1.

The remote control 200 includes a housing 204 having a grip 206. The housing 204 holds a drive gear 208, which may be actuated by a gear handle 210 to move a drive cable 212 in an exposure direction or a retraction direction. The drive cable 212 is a flexible steel cable with a helical outer winding. The helical outer winding enables the drive gear 208 to engage the drive cable 212 and advance and retract the cable 212 with a high degree of precision. The drive cable 212 engages the drive gear 208 of the remote control 200. The drive gear 208 extends the drive cable 212 (and the radiographic source 102) by driving the drive cable 212 in a first direction, and retracts the drive cable 212 (and the radiographic source 102) by driving the drive cable 212 in a second direction.

The example remote control 200 includes a control conduit 214 to cover and guide the drive cable 212 at least between the drive gear 208 and the radiographic source housing 104. In the example of FIGS. 2 and 3, the control conduit 214 is coupled to a conduit connector 216 that secures the control conduit 214 to the radiographic source housing 104 and aligns an outlet of the control conduit 214 with a corresponding inlet of the radiographic source housing 104. For example, the drive cable 212 and a source connector 218 (FIGS. 1A and 1B) may be connected to the radiographic source 102, via a pigtail connector 122 and a pigtail cable 124 (FIGS. 1A and 1B) within the radiographic source housing 104. Once connected, the conduit connector 216 can be connected to the radiographic source housing 104 and secured, and the drive cable 212 may be physically attached to the pigtail cable 124 and affixed to the radiographic source 102 via the source connector 218. In some examples, the pigtail cable 124 is made of the same or similar construction as the drive cable 212.

The example conduit connector 314 may be implemented using conventional connectors used with the Sentinel 880 Gamma Ray Source Projection system, sold by QSA Global, Inc., of Burlington, MA. However, other conduit connector styles may be used.

As illustrated in FIGS. 2 and 3, the drive cable storage drum 202 is positioned on an opposite side of the drive gear 208 from the control conduit 214. The drive cable storage drum 202 stores a portion of the drive cable 212. In operation, the drive cable storage drum 202 feeds the drive cable 212 from within the drive cable storage drum 202 to the drive gear 208 as the drive gear 208 is actuated in the feeding or forward direction (e.g., as the radiographic source 102 is exposed). Conversely, the drive cable storage drum 202 receives the drive cable 212 from the drive gear 208, and stores the drive cable 212 from within the drive cable storage drum 202, as the drive gear 208 is actuated in the retraction or reverse direction (e.g., as the radiographic source 102 is retracted or moved to the storage position from the exposed position).

The drive cable storage drum 202 may be configured to coil the drive cable 212 within the drive cable storage drum 202, and may include one or more coiling features, feeding features, anti-tangling features, and/or any other features to aid in smooth operation of the remote control 200. While the example drive cable storage drum 202 is illustrated in FIGS. 2 and 3 with a particular shape and size relative to the grip 206, other shapes may be used to increase or decrease capacity, change the weight balance based on the quantity of control cable 212 to be stored, and/or otherwise modify the use of the remote control 200.

The example control conduit 214 is coupled to the remote control housing 204 and to the conduit connector 216 via rotating connections 220. For example, the rotating connections may be a threaded connection with bearings to allow for rotation of the conduit 214, a quick-connect type of connection, and/or any other type of connection. The rotating connections 220 of the control conduit 214 reduce torsion and/or other stresses on the control conduit 214 due to rotation of the drive cable 212.

In the illustrated example, the drive cable storage drum 202 is a fixed size and in a fixed position with respect to the drive gear 208 at the end of the grip 206. For example, the drive cable storage drum 202 may be installed (e.g., via a threaded connection, a bayonet connection, a locking pin, etc.), affixed, fastened, and/or otherwise attached to the grip 206. The drive cable 212 is conducted through the grip 206 between the drive gear 208 and the drive cable storage drum 202, such as via a bore 224 through the grip 206. In other examples, the drive cable storage drum 202 may be coupled to the grip 206 or other portion of the remote control housing 204 via a storage conduit 226 (FIG. 1).

The drive cable storage drum 202 and/or the grip 206 may include a window 222 to enable the operator, maintenance personnel, and/or others to view portions of the drive cable within the drive cable storage drum 202.

The drive cable storage drum 202 further improves the ease of maintenance of the drive cable 212, such as by allowing for cleaning and/or lubrication of the drive cable 212 to occur within the drive cable storage drum 202 instead of removing the entire control cable from the remote control 200.

While FIGS. 2 and 3 illustrate a pistol-style housing, reel-style housings and/or any other type of housing may be used. Additionally or alternatively, the drive gear 208 may be replaced with a different type of actuator and/or drive interface to drive the drive cable 212. In some examples, the drive gear 208 (or other drive interface) may be automatically actuated (e.g., via a motor) instead of manually actuated via a handle.

In some examples, the drive cable 212 may be divided into a drive section and a forward cable section having a lower-friction surface. For example, the forward cable section may include an interior column constructed using one or more of a semi-rigid spring material, a spiral wound cable, a close-wound cable, and/or a compacted wire rope. The interior column may be a metal, a polymer, and/or any other material that has acceptable flexibility and is capable of linear actuation while maintaining sufficient strength in a radioactive environment for at least a threshold life span. By using a forward cable section having a lower friction, the radiographic source housing 104 may be subjected to less wear during operation.

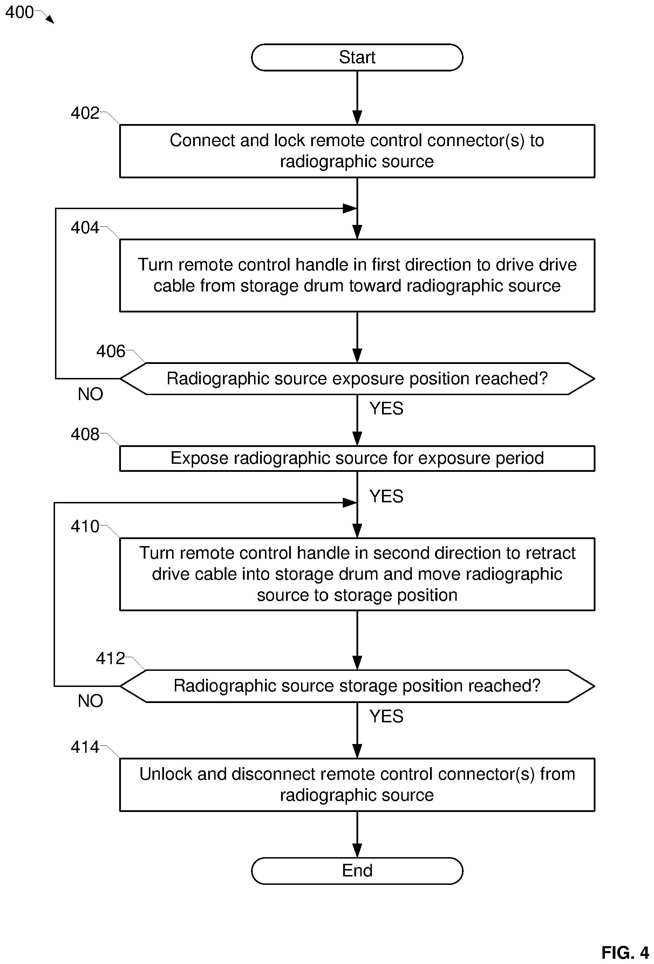

FIG. 4 is a flowchart representative of an example method 400 which may be performed to control exposure of a radiographic source 102 (FIG. 1) using the remote control 200 of FIGS. 2 and 3. While the method 400 is disclosed below with reference to certain applicable elements, local regulations may require that additional actions or steps, such as (but not limited to) confirmatory surveys of radiation levels prior to and/or after exposure of the radioactive source, be taken in conjunction with the disclosed steps.

At block 402, the conduit connector 216 and the source connector 218 are connected and locked to the radiographic source 102. For example, the source connector 218 may be connected to the pigtail connector 122, and the conduit connector 216 locks the control conduit 214 to the radiographic source housing 104. Connecting to the source housing 104 may include connecting to the shield 108 and/or to any other fixed element of the housing 104.

At block 404, the operator turns the gear handle 210 in a first direction (e.g., exposure direction) to drive the drive cable 212 from the drive cable storage drum 202 toward the radiographic source 102. The driving of the drive cable 212 from the drive cable storage drum 202 to the drive gear 208 is guided by the grip 206 and/or a storage conduit, and is guided by the control conduit 214 between the drive gear 208 and the radiographic source housing 104. As the drive cable 212 is driven, the drive cable 212 drives the radiographic source 102 from a storage position within the radiographic source housing 104 through the guide tube 114 toward a predetermined exposure position.

At block 406, the operator determines whether the radiographic source exposure position has been reached. If the exposure position has not been reached (block 406), the method returns to block 404 to continue driving the drive cable 212.

When the exposure position has been reached (block 406), at block 408 the operator exposes the radiographic source for an exposure period. The exposure period may be specified by a predetermined radiography technique or other method.

At block 410, the operator turns the gear handle 210 in a second direction (e.g., retraction direction) to retract (e.g., pull) the drive cable 212 from the radiographic source housing 104 and to pull the radiographic source 102 into a storage position in the radiographic source housing 104. As the drive cable 212 is pulled by the drive gear 208, the drive cable 212 is driven into the drive cable storage drum 202 for storage.

At block 412, the operator determines whether the radiographic source storage position has been reached. If the storage position has not been reached (e.g., full retraction) (block 406), the method returns to block 410 to continue retracting the drive cable 212.

At block 414, after the drive cable 212 and the radiographic source 102 are fully retracted, the operator unlocks the conduit connector 216 and disconnects the source connector 218 from the radiographic source 102. Full retraction refers to the source connector 218 reaching the conduit connector 216, or when the drive cable 212 is mechanically stopped from further retraction. The example method 400 then ends.

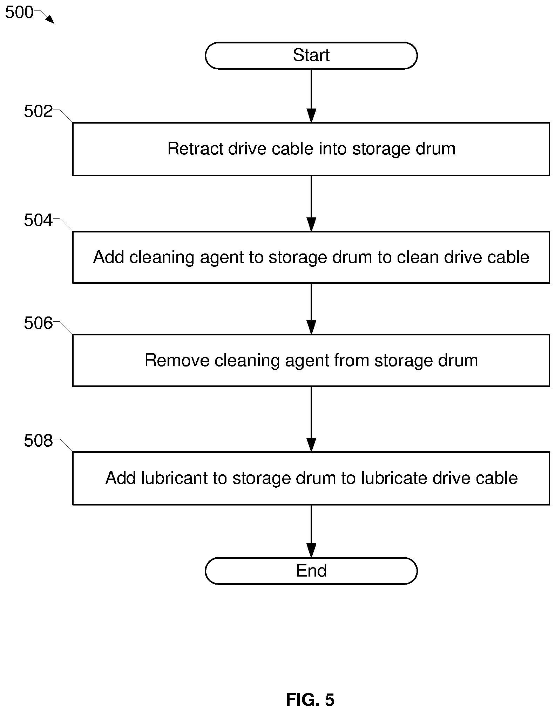

As mentioned above, at least a portion of the drive cable 212 may be cleaned and/or lubricated within the drive cable storage drum 202. FIG. 5 is a flowchart representative of an example method 500 which may be performed to clean the drive cable 212 of the remote control 200 of FIGS. 2 and 3.

At block 502, the operator retracts the drive cable 212 into the drive cable storage drum 202. In some examples, the drive cable 212 is stopped from being retracted by contact between the source connector 218 and the conduit connector 216, such that the source connector 218 is not retracted into the control conduit 214. In other examples, the source connector 218 and the conduit connector 216 may be configured and/or adjustable to enable the source connector 218 to be retracted into and/or past the control conduit 214, such that an additional length of the drive cable 212 can be retracted into the storage drum 202. In some examples, the drive cable storage drum 202 may be removed or disconnected from the grip 206 and/or from a storage conduit to allow access to an opening in the drive cable storage drum 202.

At block 504, a cleaning agent is added to the drive cable storage drum 202 to clean the drive cable 212. For example, the cleaning agent may include a degreaser or solvent selected to remove contaminants and/or used lubricant from the drive cable 212. The drive cable storage drum 202 may be agitated, and/or a brush or other cleaning device inserted, to more quickly and/or thoroughly clean the drive cable 212 with the cleaning agent. The drive cable 212 may also be soaked in the cleaning agent within the drive cable storage drum 202 for a period of time.

At block 506, the operator removes the cleaning agent from the drive cable storage drum 202. For example, the operator may pour out liquid cleaning agent from an opening in the drive cable storage drum 202 and/or blow out non-liquid cleaning agents using compressed gas.

At block 508, the operator adds lubricant to the storage drum to lubricate the drive cable 212. The drive cable storage drum 202 may be agitated, and/or a brush or other cleaning device inserted, to more quickly and/or thoroughly disperse the lubricant to the drive cable 212. The example method 500 then ends.

As utilized herein, "and/or" means any one or more of the items in the list joined by "and/or". As an example, "x and/or y" means any element of the three-element set {(x), (y), (x, y)}. In other words, "x and/or y" means "one or both of x and y". As another example, "x, y, and/or z" means any element of the seven-element set {(x), (y), (z), (x, y), (x, z), (y, z), (x, y, z)}. In other words, "x, y and/or z" means "one or more of x, y and z". As utilized herein, the term "exemplary" means serving as a non-limiting example, instance, or illustration. As utilized herein, the terms "e.g.," and "for example" set off lists of one or more non-limiting examples, instances, or illustrations. As utilized herein, circuitry is "operable" to perform a function whenever the circuitry comprises the necessary hardware and code (if any is necessary) to perform the function, regardless of whether performance of the function is disabled or not enabled (e.g., by a user-configurable setting, factory trim, etc.).

While the present method and/or system has been described with reference to certain implementations, it will be understood by those skilled in the art that various changes may be made and equivalents may be substituted without departing from the scope of the present method and/or system. For example, block and/or components of disclosed examples may be combined, divided, re-arranged, and/or otherwise modified. In addition, many modifications may be made to adapt a particular situation or material to the teachings of the present disclosure without departing from its scope. Therefore, the present method and/or system are not limited to the particular implementations disclosed. Instead, the present method and/or system will include all implementations falling within the scope of the appended claims, both literally and under the doctrine of equivalents.

Aspects of the disclosure are provided in the following numbered clauses:
Clause 1. A remote control for a radiographic source, the remote control comprising:
   a drive cable configured to detachably couple to a radiographic source via a connector;
   a drive gear configured to advance the drive cable in a first direction toward the connector and to retract the drive cable in a second direction away from the connector;
   a control conduit configured to guide a portion of the drive cable located between the drive gear and a radiographic source housing; and
   a drive cable storage drum on an opposite side of the drive gear from the control conduit, and configured to store a portion of the drive cable.
Clause 2. The remote control as defined in clause 1, wherein the drive cable storage drum is a fixed size and a fixed position with respect to the drive gear.
Clause 3. The remote control as defined in clause 1, wherein the drive cable storage drum is configured to coil the drive cable within the drive cable storage drum as the drive cable is retracted by the drive gear.
Clause 4. The remote control as defined in clause 1, wherein the drive cable storage drum comprises a window configured to enable viewing of at least a portion of the drive cable within the drive cable storage drum.
Clause 5. The remote control as defined in clause 1, further comprising a drive gear housing configured to house the drive gear, the drive gear housing having a grip.
Clause 6. The remote control as defined in clause 5, wherein the drive cable storage drum is coupled to the drive gear housing via the grip.
Clause 7. The remote control as defined in clause 1, further comprising a conduit connector configured to secure the control conduit to the radiographic source housing.
Clause 8. The remote control as defined in clause 1, further comprising a motor configured to actuate the drive gear.
Clause 9. The remote control as defined in clause 1, further comprising a handle configured to actuate the drive gear.
Clause 10. The remote control as defined in clause 1, further comprising a housing configured to house the drive gear, wherein the control conduit is configured to rotate with respect to the housing.
Clause 11. The remote control as defined in clause 1, further comprising a storage conduit positioned between the drive gear and the drive cable storage drum.
Clause 12. A method to actuate a radiographic source, the method comprising:
   actuating a remote control coupled to a radiographic source in a first direction to extend a portion of a source cable of the remote control from a storage drum toward the radiographic source; and
   actuating the remote control in a second direction to retract a portion of the source cable of the remote control into the storage drum.
Clause 13. The method as defined in clause 13, further comprising cleaning at least a portion of the source cable in the storage drum.
Clause 14. The method as defined in clause 10, further comprising connecting the source cable to the radiographic source and locking a conduit for the source cable to a housing of the radiographic source via a connector prior to extending the portion of the source cable.
Clause 15. The method as defined in clause 12, further comprising unlocking the conduit and detaching the source cable from the radiographic source after fully retracting the source cable.
Clause 16. A method to clean a remote control for a radiographic source, the method comprising:
   retracting a portion of a source cable of a remote control for a radiographic source into a storage drum attached to the remote control; and
   adding a cleaning agent to the storage drum to clean the portion of the source cable within the storage drum.
Clause 17. The method as defined in clause 16, wherein the cleaning agent comprises a solvent.
Clause 18. The method as defined in clause 16, further comprising removing the cleaning agent from the storage drum.
Clause 19. The method as defined in clause 18, further comprising adding a lubricant to the storage drum to lubricate the source cable.
Clause 20. The method as defined in clause 16, wherein the retracting of the portion of the source cable comprises turning a handle of the remote control in a retraction direction.

## Claims

1. A remote control for a radiographic source, the remote control comprising:
a drive cable configured to detachably couple to a radiographic source via a connector;
a drive gear configured to advance the drive cable in a first direction toward the connector and to retract the drive cable in a second direction away from the connector;
a control conduit configured to guide a portion of the drive cable located between the drive gear and a radiographic source housing; and
a drive cable storage drum on an opposite side of the drive gear from the control conduit, and configured to store a portion of the drive cable.

2. The remote control as defined in claim 1, wherein the drive cable storage drum is a fixed size and a fixed position with respect to the drive gear, and optionally wherein the drive cable storage drum is configured to coil the drive cable within the drive cable storage drum as the drive cable is retracted by the drive gear.

3. The remote control as defined in claim 1, wherein the drive cable storage drum comprises a window configured to enable viewing of at least a portion of the drive cable within the drive cable storage drum.

4. The remote control as defined in claim 1, further comprising a drive gear housing configured to house the drive gear, the drive gear housing having a grip, optionally wherein the drive cable storage drum is coupled to the drive gear housing via the grip.

5. The remote control as defined in claim 1, further comprising a conduit connector configured to secure the control conduit to the radiographic source housing.

6. The remote control as defined in claim 1, further comprising a motor configured to actuate the drive gear, and optionally further comprising a handle configured to actuate the drive gear.

7. The remote control as defined in claim 1, further comprising a housing configured to house the drive gear, wherein the control conduit is configured to rotate with respect to the housing, and optionally wherein the remote control further comprises a storage conduit positioned between the drive gear and the drive cable storage drum.

8. A method to actuate a radiographic source, the method comprising:
actuating a remote control coupled to a radiographic source in a first direction to extend a portion of a source cable of the remote control from a storage drum toward the radiographic source; and
actuating the remote control in a second direction to retract a portion of the source cable of the remote control into the storage drum.

9. The method as defined in claim 8, further comprising cleaning at least a portion of the source cable in the storage drum.

10. The method as defined in claim 8, further comprising connecting the source cable to the radiographic source and locking a conduit for the source cable to a housing of the radiographic source via a connector prior to extending the portion of the source cable.

11. The method as defined in claim 8, further comprising unlocking the conduit and detaching the source cable from the radiographic source after fully retracting the source cable.

12. A method to clean a remote control for a radiographic source, the method comprising:
retracting a portion of a source cable of a remote control for a radiographic source into a storage drum attached to the remote control; and
adding a cleaning agent to the storage drum to clean the portion of the source cable within the storage drum.

13. The method as defined in claim 12, wherein the cleaning agent comprises a solvent.

14. The method as defined in claim 12, further comprising removing the cleaning agent from the storage drum, optionally wherein the method further comprises adding a lubricant to the storage drum to lubricate the source cable.

15. The method as defined in claim 12, wherein the retracting of the portion of the source cable comprises turning a handle of the remote control in a retraction direction.
